# EUROPEAN PATENT APPLICATION

(11) **EP 4 814 904 A2**
(43) Date of publication of application: **30.09.2026**
(21) Application number: 26199419.8
(22) Date of filing: 12.01.2022
(51) Int. Cl.: G06V 20/69

(54) **SYSTEMS AND METHODS FOR NON-INVASIVE PREIMPLANTATION EMBRYO GENETIC SCREENING**

(30) Priority: 12.01.2021 US 202163136382 P
(62) Document number(s) of the earlier application(s) in accordance with Art. 76 EPC: 22738866.7 / 4 278 353
(71) Applicant: Trio Fertility Research Inc., Toronto, Ontario M5G 2K4 (CA)
(72) Inventor: CASPER, Robert, Toronto, Ontario, M5G 2K4 (CA); MERIANO, James, Toronto, Ontario, M5G 2K4 (CA); MERHAV, Uri, Toronto, Ontario, M5G 2K4 (CA)
(74) Representative: Tombling, Adrian George

(57) **Abstract**

Embryo genetic screening is performed by optical inspection, such as receiving, from an image sensor, image data representing emerging polarized light that has traversed a specimen, determining birefringence properties of the specimen based at least in part on the image data, generating a polarized light image representative of the specimen based at least in part on the birefringence properties, classifying features of the polarized light image using a classifier, identifying features of the polarized light image as mitotic spindles, determining mitotic activity of the specimen based at least in part on the identified mitotic spindles, and predicting a ploidy status of the specimen based on the mitotic activity.

## Description

### CROSS-REFERENCE TO RELATED APPLICATIONS

This application claims all benefit, including priority of U.S. Provisional Patent Application No. 63/136,382, filed January 12, 2021, the entire contents of which are incorporated herein by reference.

### FIELD

This disclosure relates to systems and methods for genetic screening of embryos, such as for use with in vitro fertilization.

### BACKGROUND

In recent years preimplantation genetic testing for aneuploidy (PGT-A, formerly known as preimplantation genetic screening or PGS) has become a very common tool in assisted reproduction. It is estimated that up to 20% of clinics in the USA offer PGT-A to all patients undergoing IVF.

PGT-A is a chromosomal test for embryos created in the IVF process. It involves a biopsy of trophectoderm (TE) cells done on each of the created embryos that reaches the blastocyst stage, amplification of the DNA extracted from the cells and performing next generation sequencing (NGS) to identify numerical chromosomal abnormalities in the cells [1,2].

The theoretical advantage of choosing only euploid embryos is to increase the chance of live birth per embryo transfer thus reducing the time to pregnancy and to lower the chance of miscarriage. One of the disadvantages of PGT-A is that it is an invasive procedure with possible damage to the embryo since the biopsy reduces the number of cells present in the TE [3]. There is also a risk of DNA contamination or amplification errors. Further, allowing time for PGT-A may require that embryos be frozen prior to transfer.

In PGT-A, the chromosomal testing is done on the TE cells that become the placenta and this result may not reflect the chromosomal makeup of the inner cell mass (ICM) that becomes the baby. It appears that about 60% of day 3 embryos contain both chromosomally normal (euploid) and chromosomally abnormal (aneuploid) cells, a condition termed mosaicism. A single TE biopsy may by chance pick up one or the other type of cell or a combination of both from the TE without sampling the ICM. Hence the accuracy of PGT-A has been questioned.

There remains a need for less invasive and more accurate tests for the chromosomal make-up of an embryo.

### SUMMARY

According to an aspect, there is provided a computer-implemented system for classifying ploidy status. The system includes: a processor; and a memory in communication with the processor, the memory storing instructions that, when executed by the processor, cause the processor to: receive polarized light image data reflective of a mammal embryo specimen; present the polarized light image data to a convolutional neural network (CNN) trained to classify specimens according to a ploidy status; and generate with the CNN a classification metric reflective of a likelihood of the ploidy status.

In some embodiments, the ploidy status includes at least one of aneuploidy, mosaicism, or euploidy.

In some embodiments, the classification metric is received from a classification head of the CNN, and the CNN further includes a segmentation head configured to predict, for a given pixel in the image data, whether the pixel represents a particular embryo feature.

In some embodiments, the CNN is trained using a loss function that includes a classification loss for the classification head, and a segmentation loss for the segmentation head, and the loss function includes a relative weight of the classification loss and segmentation loss.

In some embodiments, the particular of embryo feature is an inner cell mass, a trophectoderm, or a zona.

In some embodiments, the polarized light image data includes a frame reflecting a particular imaged layer of the mammal embryo specimen.

In some embodiments, the polarized light image data includes a plurality of frames, each reflecting a particular imaged layer of the mammal embryo specimen.

In some embodiments, the CNN includes an inner layer configured to produce a plurality of representation vectors, each corresponding to one of the plurality of frames, and the representation vectors are provided to a 1D convolutional layer of the CNN.

In some embodiments, the CNN is a 3D convolutional neural network and the polarized image data is organized as a volume including the plurality of frames.

In some embodiments, the instructions, when executed by the processor cause the processor to: provide metadata of the mammal embryo specimen to the CNN.

In some embodiments, the metadata is provided to an inner layer of the CNN.

In some embodiments, the metadata is concatenated to the output of a layer preceding the inner layer.

In some embodiments, the instructions, when executed by the processor cause the processor to: maintain a look-up table for mapping values of the metadata to values trained with the CNN.

In some embodiments, wherein the metadata includes a patient's age.

In some embodiments, the mammal is a human.

In some embodiments, the instructions, when executed by the processor cause the processor to generate the polarized light image data upon determining birefringence properties of the mammal embryo specimen.

According to another aspect, there is provided a computer-implemented method for classifying ploidy status. The method includes: receiving polarized light image data reflective of a mammal embryo specimen; presenting the polarized light image data to a convolutional neural network (CNN) trained to classify according to a ploidy status; and generating with the CNN a classification metric reflective of a likelihood of the ploidy status
According to another aspect, there is provided a computer-implemented system comprising: an image sensor; a processor in communication with the image sensor; and a memory in communication with the processor, the memory storing instructions that, when executed by the processor cause the processor to: receive, from the image sensor, image data representing emerging polarized light that has traversed a specimen; determine birefringence properties of the specimen based at least in part on the image data; generate a polarized light image representative of the specimen based at least in part on the birefringence properties; classify features of the polarized light image using a classifier; identify features of the polarized light image as mitotic spindles; determine mitotic activity of the specimen based at least in part on the identified mitotic spindles; and predict a ploidy status of the specimen based on the mitotic activity.

In some embodiments, the memory stores further instructions that, when executed by the processor cause the processor to: determine whether the mitotic activity is below a predetermined threshold, and when the mitotic activity is below the predetermined threshold the ploidy status of the specimen is predicted to be euploid.

In some embodiments, the mitotic activity of the specimen is determined based at least in part on a number of the identified mitotic spindles.

In some embodiments, the memory stores further instructions that, when executed by the processor cause the processor to: determine geometric shapes of the identified mitotic spindles; and the mitotic activity of the specimen is determined based at least in part on the geometric shapes of the identified mitotic spindles.

In some embodiments, the memory stores further instructions that, when executed by the processor cause the processor to: identify features of the polarized light image as an inner cell mass (ICM) and a trophectoderm (TE); determine locations of the identified mitotic spindles as in the ICM or in the TE; and the mitotic activity of the specimen is determined based at least in part on the locations of the identified mitotic spindles.

In some embodiments, the specimen is from a mammal embryo.

In some embodiments, the mammal is a human.

According to another aspect, there is provided a method of selecting for implantation an embryo of a mammal egg fertilized in vitro comprising: quantifying mitotic activity in the trophectoderm (TE) and/or the inner cell mass (ICM) of an embryo based on optical inspection thereof; and selecting an embryo that has mitotic activity below a predetermined threshold.

In some embodiments, an embryo having mitotic activity below the predetermined threshold is predicted to be euploid.

In some embodiments, the optical inspection comprises imaging the embryo using polarized light with a microscope equipped with a retardance filter, wherein a detected degree of retardance corresponds to the mitotic activity.

In some embodiments, the mitotic activity is based at least in part on the number of mitotic spindles identified in the ICM.

In some embodiments, the mitotic activity is based at least in part on the number of mitotic spindles identified in the TE.

In some embodiments, the predetermined threshold is zero mitotic activity in the ICM.

In some embodiments, the mitotic activity is based at least in part on the total number of mitotic spindles in the TE and ICM.

In some embodiments, the mitotic activity is a weighted average of the number of mitotic spindles in the ICM and the total number of mitotic spindles in the TE and ICM.

In some embodiments, the optical inspection is performed between 2 days and 10 days after fertilization of the specimen.

In some embodiments, the optical inspection is performed at 5 days after fertilization of the specimen.

In some embodiments, the method further comprises implanting the selected embryo.

In some embodiments, the embryo is not frozen prior to implantation.

In some embodiments, the mammal is a human.

In some embodiments, the threshold is determined by a patient.

According to a futher aspect, there is provided a method for optimizing the likelihood of a live birth after implantation of a human egg fertilized in vitro comprising: performing a method as disclosed herein on a plurality of embryos developed after in vitro fertilization and selecting an embryo that is predicted to be euploid for implantation.

In some embodiments, the method further comprises selecting the embryo having the highest probability of being euploid for implantation.

According to yet another aspect, there is provided a tool for assisting a human patient undergoing in vitro fertilization treatment in deciding whether to proceed with implantation of an embryo resulting from a human egg fertilized in vitro, the tool comprising an indicia of the probability of the embryo being euploidy and/or aneuploidy based on the number of mitotic spindles in the TE and/or the ICM as observed by an optical inspection method at the blastocyst stage.

Other features will become apparent from the drawings in conjunction with the following description.

### BRIEF DESCRIPTION OF DRAWINGS

In the figures which illustrate example embodiments,
**FIG. 1** is a schematic diagram of a system for spindle imaging and ploidy prediction, according to an embodiment;
**FIG. 2A** illustrates a light microscope image of an embryo specimen, according to an embodiment;
**FIG. 2B** illustrates a polarized light image of an embryo specimen, according to an embodiment;
**FIG. 3** is a flow chart of a method for predicting ploidy status, according to an embodiment;
**FIG. 4** is a flow chart of a method for detecting mitotic spindles, according to an embodiment;
**FIG. 5** is a schematic diagram of a computing device for processing polarized light image data, according to an embodiment;
**FIG. 6A** and **6B** each is a schematic diagram of a deep machine learning model implemented at the computing device of **FIG. 5****,** according to respective embodiments;
**FIG. 7A** illustrates a polarized light image of an embryo specimen, according to an embodiment;
**FIG 7B** illustrates the polarized light image of **FIG. 7A** with feature segmentation, according to an embodiment;
**FIG. 7C** and **FIG. 7D** each illustrate a respective polarized light image with feature segmentation, according to an embodiment;
**FIG. 8** is a schematic diagram showing the provision of metadata to a deep machine learning model, according to an embodiment,
**FIG. 9** is a flow chart of a method for classifying ploidy status, according to an embodiment; and
**FIG. 10** is a block diagram of example hardware and software components of a computing device for predicting ploidy, according to an embodiment;

### DETAILED DESCRIPTION

Provided herein are systems and methods for determining the ploidy status of embryos that are less invasive than the current standard PGT-A. Further, these methods can yield faster results than PGT-A, and so may avoid the need for embryo cryopreservation.

These systems and methods can also improve accuracy by better predicting the ploidy status of babies from mosaic embryos. Euploid cells of mosaic embryos may remain while aneuploid cells may disappear during development, leading to chromosomally normal babies. Experimental evidence for such a mechanism comes from a mouse model in which chimeras (mixtures) of euploid cells and aneuploid cells showed selective programmed cell death (apoptosis) of aneuploid cells in the inner cell mass (ICM) and proliferative defects of aneuploid cells in the trophectoderm (TE), leading to a progressive depletion of aneuploid cells from the blastocyst stage onward [4].

The true incidence of mosaicism in human blastocyst embryos is unknown.

Studies of the current standard for genetic screening, PGT-A, have revealed a risk of both false positives for aneuploidy, which can lead to healthy embryos not being selected for transplantation, and false negatives. For example, a prospective study of transferred embryos without disclosing the biopsy result found that 3 of 46 PGT-A screened aneuploidy blastocysts resulted in healthy live births [5]. Another study showed that up to 29% of embryos diagnosed as aneuploid by PGT-A were actually normal when retested [6].

A study done by Victor et. al. [7] on human embryos in order to determine the implantation potential of mosaic embryos revealed that a large proportion of mosaic as well as aneuploid blastocysts displayed medium or high levels of mitosis and apoptosis in the TE compared with euploid blastocysts. In the ICM, some mosaic and aneuploid blastocysts displayed minimal/low, medium or high levels of mitosis and apoptosis while euploid embryos had virtually no mitotic activity in the ICM.

In a method of the present invention, an assessment of the mitotic activity of the ICM and/or TE during the blastocyst stage is performed to obtain an indication of the chromosomal status of an embryo using non-invasive optical techniques.

Systems and methods disclosed herein may be used to assess mitotic activity of ICM and TE, for example, during a blastocyst stage of an embryo to predict the chromosomal status of the embryo.

Mitotic activity can be assessed non-invasively by imaging the embryo using polarized light with a microscope equipped with a retardance filter. By recording the degree of retardance in the ICM and TE, it may be possible to determine the number of mitotic spindles since mitotic spindles have high retardance and appear as bright areas. In this way, mitotic spindles may be visualized during embryo development. The more mitotic spindles that are observed, the higher the mitotic activity of the embryo. The retardance can also be quantitated using image analysis to get a numerical value. Polarized light images of the embryo can also be analyzed by artificial intelligence (AI) algorithms using machine learning and neural networks, such as those disclosed herein. An AI algorithm may also be suitable for analysis of digital video capture of moving focal plane microscopic imaging of an entire blastocyst.

Polarized light used for imaging as disclosed herein may be a higher setting visible microscope light, to which an embryo or other specimen may be exposed for a very short time. Use to-date has not indicated that short exposure to visible light harms embryos. The polarized light used may be a smaller spectrum range than all visible wavelengths, since the light is polarized into a specific longer visible wavelength.

Embryo selection, for example, for implantation, can be based on a retardance that falls below a predetermined threshold. The predetermined threshold may be set at a level below which the embryo is predicted to be euploid, e.g. it may be set at a level where the likelihood of the embryo being euploid is > 50 %, > 80%, > 90%, > 95% or > 99%.

While the systems and methods of the present invention may be used to assist human reproduction, they may also be used in other mammalian species, including, without limitation, livestock, such as cattle and equine species, companion animals, including intentionally bred dogs and cats, as well as non-domesticated mammals, where in vitro fertilization may be used to assist in preservation efforts for endangered species.

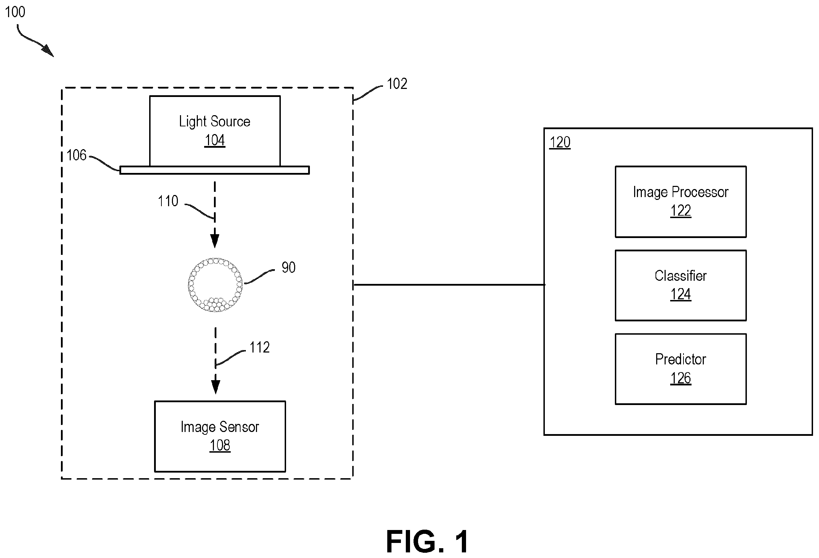

**FIG.** 1 is a schematic diagram of a system 100 for optical inspection, in particular spindle imaging, and ploidy prediction of a specimen, according to an embodiment. Spindle imaging may be used to assess mitotic activity, and thus used to determine ploidy status based at least in part on degree of mitotic activity.

In some embodiments, system 100 may incorporate elements of an Oosight^{®} imaging system.

As shown in **FIG. 1****,** in some embodiments, system 100 includes a microscope 102 in communication, over a suitable communication link, with a computing device 120 for imaging a specimen 90.

System 100 includes microscope 102 such as an inverted microscope including a light source 104, a retardance filter 106 and an image sensor 108 as illustrated in **FIG. 1****.** Retardance filter 106 may alter polarization of light travelling through it, from light source 104, to generate polarized incident light 110. Image sensor 108 can be any suitable image capture component, for example a digital camera (with still and/or video recording capabilities) and can include CMOS or CCD sensors.

System 100 may be used to image a specimen 90 such as an oocyte or embryo, in particular, the inner cell mass (ICM) and outer cells or trophectoderm (TE) of an embryo. Polarization of light in the direction of an oocyte or embryo allows spindle(s) to be visible, for example, to an embryologist during intracytoplasmic sperm injection (ICSI) so that the spindle structure can be avoided during the procedure. Polarized light is harmless to an oocyte or embryo and the polarized light is refracted by the spindle so that it is visible, as described herein.

In some embodiments, specimen 90 may be a cryopreserved embryo or has been previously cryopreserved. In some embodiments, specimen 90 has not been cryopreserved.

The specific timing of optical inspection of the embryo may be optimized for the practice of the method in different species, although the optical inspection will suitably occur within 10 days after fertilization of the oocyte. Optical inspection of a specimen 90 by system 100 may be performed on a specimen 90 that is between 2 days and 10 days after fertilization, in an example, between 3 days and 7 days, and system 100 may receive as input an age of specimen 90, such as a number of days following fertilization. In practice, human embryos in culture for clinical use may be used at age 7 days or younger.

As illustrated in **FIG. 1****,** as incident light 110 traverses specimen 90, emerging light 112 is captured by image sensor 108 and measured to generate a polarized light image of specimen 90, in whole or in part, represented by image data.

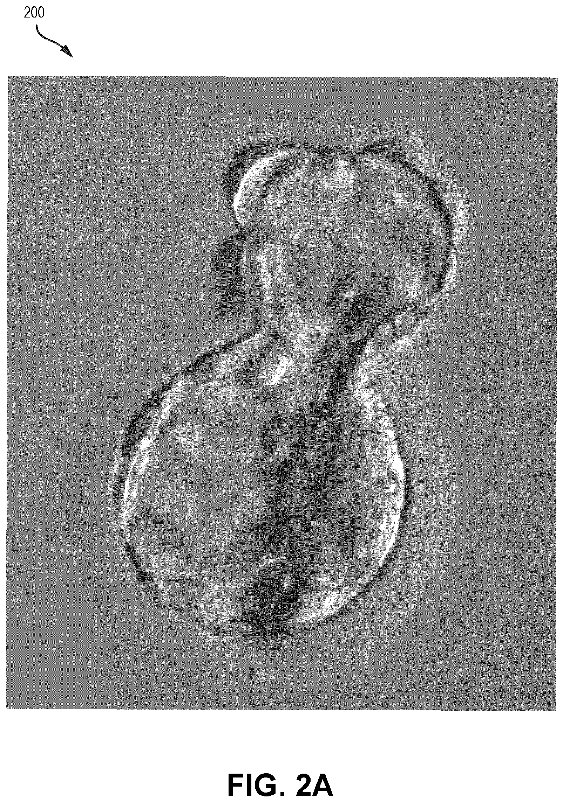

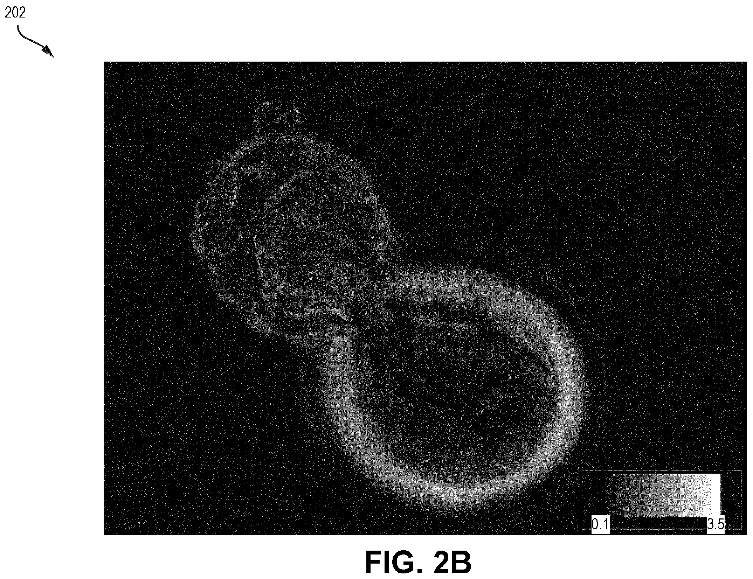

**FIG. 2A** illustrates a graphic representation of a standard light microscope image 500 of an imaged embryo specimen 90, according to an embodiment. **FIG. 2B** illustrates a graphic representation of a polarized light image 502 of an imaged embryo specimen 90, showing mitotic spindles, according to an embodiment

In some embodiments, image data detected by image sensor 108 can include video data, in particular, a sequence of individual video frames or images captured over time. In an example, using an optical lens or assembly of lenses to focus light on image sensor 108, focus on a specimen 90 can be varied, bringing different layers of specimen 90 into focus, as video is captured.

Image data detected by image sensor 108 can be sent to computing device 120 for processing.

Computing device 120 is configured to process polarized light image data that can be used to automatically predict ploidy status in a specimen 90.

As shown in **FIG. 1****,** system 100 can include an image processor 122, a classifier 124 and a predictor 126.

While illustrated on computing device 120, it is understood that one or more of image processor 122, classifier 124 and predictor 126 may be implemented on microscope 102 or any other suitable processing device including software and/or hardware.

Image processor 122 may be configured to receive image data from microscope 102 and determine birefringence of specimen 90 by measuring the change in a polarized beam of light as it traverses specimen 90 (for example, a phase shift light detected by image sensor 108). The birefringence properties can be used to generate a polarized light image representative of specimen 90.

Retardance can be determined based on a magnitude of birefringence as a function of sample thickness - on a per pixel basis in an acquired polarized light image. In some embodiments, azimuth can also be measured on a per pixel basis in an acquired polarized light image.

Image processor 122 may process image data frames from video data captured over a period of time, which may reflect differing focus of specimen 90, with each image data frame reflecting a different layer of specimen 90.

In some embodiments, image processor 122 can be implemented using suitable machine learning techniques.

Classifier 124 can identify features, such as number or shape of mitotic spindles, in image data. Classifier 124 may be any suitable classification model. In some embodiments, classifier 124 is a convolutional neural network (CNN) trained using suitable training data and techniques and feature extraction and feature selection is performed to generate features from the training data. Classifier 124 can be implemented using other suitable machine learning techniques or neural networks. It will be understood that other suitable image recognition or object detection techniques may be implemented to detect instances of features such as mitotic spindles from image data.

Classifier 124 can be configured to identify and distinguish between an inner cell mass (ICM) and outer cells (trophectoderm (TE)) in image data. In some embodiments, classifier 124 can be configured to identify further regions such as, for example, a zona, and distinguish such regions from ICM, TE, etc.

Classifier 124 can be configured to classify (such as by way of a probability value) features of image data for identifying mitotic spindles, determining a number of the mitotic spindles, size of the mitotic spindles, shape or geometry of the mitotic spindles, and position/location of the mitotic spindles. In an example, mitotic spindles may be classified as located in either an ICM or the outer cells (such as TE).

In some embodiments, classifier 124 can classify features with a percentage likelihood, and an output classification may be based at least in part on whether that likelihood meets a threshold.

Classification by classifier 124 may be based at least in part on an age (such as the number of days following fertilization) of the specimen 90 in the image data. In an example, an earlier embryo (for example, at day 3) may have less cells, and thus different features which may be classified differently. There may also be an age of specifimen 90 that corresponds with a peak mitotic activity as an embryo grows, which classifier 124 can take into account in classification. Thresholds for feature classification may be based at least in part on the age of specimen 90.

Predictor 126 may predict a ploidy status of specimen 90 based on a degree of determined mitotic activity, based at least in part on a number of mitotic spindles, or other features such as size, shape and position/location of mitotic spindles, detected in image data of a specimen 90.

Mitotic activity may be based at least in part on a number of mitotic spindles. Furthermore, since the number of cells in an ICM and in a TE will change depending on the age of the embryo and the grading of the embryo which is determined by the expansion of the blastocyst, mitotic activity may be based on the number and quality of cells in the ICM and the number and quality of cells in the TE.

In some embodiments, mitotic activity may be determined based at least in part on a ratio of mitotic spindles to cells in the ICM or in the TE, or a ratio between retardance or number of mitotic spindles in ICM and number of mitotic spindles in TE (an ICM:TE spindle ratio) or in another aspect.

Mitotic activity may be based on a degree of retardance determined by image processor 122, which may also reflect a degree of brightness and also be dependent on a number of other variables.

In some embodiments, the shape or position of the retardance, or the shape and position of detected mitotic spindles, can be used to determine mitotic activity.

Predictor 126 may be configured for predictions such as chromosomal abnormality more generally, or generating a risk probability of a miscarriage, in an example, based at least in part on features identified and classified in image data of a specimen 90.

In an example, predictor 126 can learn to associate a number of mitotic spindles, or particular shape geometries of mitotic spindles with different conditions such as mitotic activity and ploidy status.

In some embodiments, predictor 126 can generate predictions as a percentage likelihood of a condition occurring, and a prediction may be based at least in part on whether that likelihood meets a threshold.

Prediction by predictor 126 may be based at least in part on an age (such as the number of days following fertilization) of the specimen 90 in the image data. In an example, an earlier embryo (for example, at day 3) may have less cells, and thus depending on age, a mitotic activity can be used to predict different outcomes. There may also be an age of specimen 90 that corresponds with a peak mitotic activity as an embryo grows, which predictor 126 can take into account in prediction. Thresholds for mitotic activity may be based at least in part on the age of specimen 90.

In some embodiments, predictor 126 can be implemented using suitable machine learning techniques.

Machine learning models as disclosed herein, such as image processor 122, classifier 124, and predictor 126, may be trained using suitable labeled training data and a suitable optimization process to minimize a loss function (e.g., a cross-entropy loss).

In an example, for predictor 126, optimization can include minimizing the "loss" or the error as between the prediction (of the ploidy status) and the ground truth (of the ploidy status). Optimization may be performed using a suitable technique such as a stochastic gradient descent optimization algorithm.

The parameters of machine learning models can be continuously tuned to improve accuracy of the model, for example by gathering suitable data to enhance data fed to the model.

In an example, with input data to the machine learning model being image data, such as that described above, image data may be re-assessed based on outcomes, such as chemical pregnancy, confirmed viable pregnancy, results of later genetic screening (e.g., amniocentesis, noninvasive prenatal tests, etc.), live births, genetic state of live births (or any subset thereof), or any other suitable outcome. Such outcomes can be used to form enhanced data as input to a machine learning model, such that the model refines its analysis of the image data and becomes more discriminating over time.

In some embodiments, training and tuning of model parameters may be performed using a ground truth, such as genetic testing (such as by way of biopsy), a confirmed pregnancy, and a confirmed diagnosis of down syndrome after a live birth, and can also be performed based at least in part on probative but not conclusive evidence.

Data may be given a certain weight or certain model parameters weighted depending on observed outcomes, for example, from quite low in the case of a pregnancy that results in an early miscarriage to 100% for confirmed status at live birth.

As an example, chromosomal abnormalities can be associated with miscarriage, but miscarried embryos may not be tested for genetic abnormalities. The miscarriage itself, however, may be relevant to assessing image data. An image, or features thereof, such as those identified by classifier 124, initially identified as a threshold for predicting a risk of miscarriage may result in more than normal miscarriages, suggesting that this threshold is indeed an appropriate one for predicting chromosomal abnormalities.

In some embodiments, outcomes of machine learning models disclosed herein may be compared to results of embryos donated for research purposes to refine learning.

It will be appreciated that machine learning refinement may occur at different model stages and at different time points. In an example, feedback can be used to refine models after deployment.

Traditional pre-birth (in utero) genetic testing may not be a hundred percent accurate, and can generate a percentage probability of certain genetic conditions. Conveniently, embodiments as disclosed herein may provide increased accuracy of prediction.

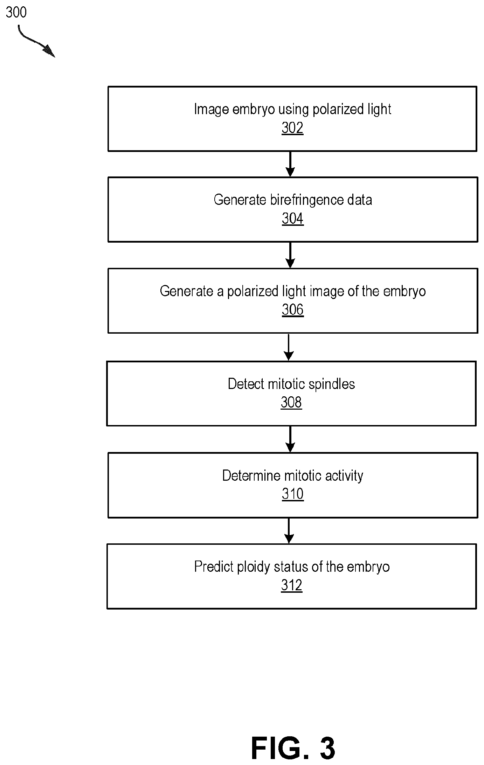

**FIG. 3** is a flow chart of a method 300 for predicting ploidy status, according to an embodiment. The steps are provided for illustrative purposes. Variations of the steps, omission or substitution of various steps, or additional steps may be considered.

At block 302, specimen 90 such as an embryo is imaged using polarized light. Specimen 90 is illuminated with polarized incident light 110 from light source 104.

At block 304, the emerging light 112 changed from traversing specimen 90 is measured to determine birefringence properties of specimen 90 by image processor 122.

At block 306, image processor 122 generates a polarized light image of specimen 90 based on the birefringence data.

At block 308, mitotic spindles in the polarized light image are detected by classifier 124. Mitotic spindles may be identified in the image using method 300 for detecting mitotic spindles, as described in further detail below.

In some embodiments, classifier 124 determines geometric shapes of the identified mitotic spindles.

In some embodiments, classifier 124 identifies features of the polarized light image as an inner cell mass (ICM) and a trophectoderm (TE) and determines locations of the identified mitotic spindles as in the ICM or in the TE.

At block 310, predictor 126 determines mitotic activity, based at least in part on the number of mitotic spindles detected.

In some embodiments, the mitotic activity of specimen 90 is determined based at least in part on a number of the identified mitotic spindles.

In some embodiments, the mitotic activity of specimen 90 is determined based at least in part on the geometric shapes of the identified mitotic spindles.

In some embodiments, the mitotic activity of the specimen is determined based at least in part on the locations of the identified mitotic spindles (for example, in the ICM or TE).

At block 312, predictor 126 predicts a ploidy status of specimen 90, based at least in part on the degree of mitotic activity that has been detected. Predicted ploidy status can include, for example, aneuploidy (addition or loss of one or more chromosomes of a set), monoploidy (loss of a set of chromosomes), euploidy (duplication of a set of chromosomes), or mosaicism.

In some embodiments, predictor 126 determines whether the mitotic activity is below a predetermined threshold, and when the mitotic activity is below the predetermined threshold the ploidy status of the specimen 90 is predicted to be euploidy.

It should be understood that blocks 302 to 312 may be performed in a different sequence or in an interleaved or iterative manner.

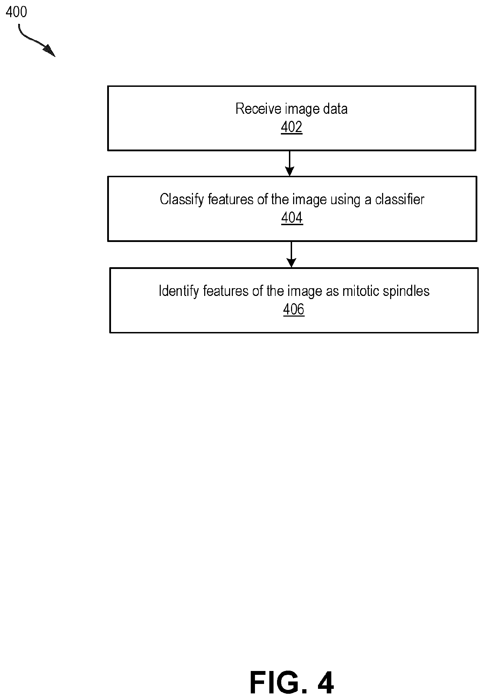

**FIG. 4** is a flow chart of a method 400 for detecting mitotic spindles using a classifier or pattern recognition model such as classifier 124, according to an embodiment. The steps are provided for illustrative purposes. Variations of the steps, omission or substitution of various steps, or additional steps may be considered.

At block 402, input image data, for example, a polarized light image, is received.

At block 404, features of the polarized light image are classified.

At block 406, features of the image are identified as mitotic spindles.

It should be understood that blocks may be performed in a different sequence or in an interleaved or iterative manner.

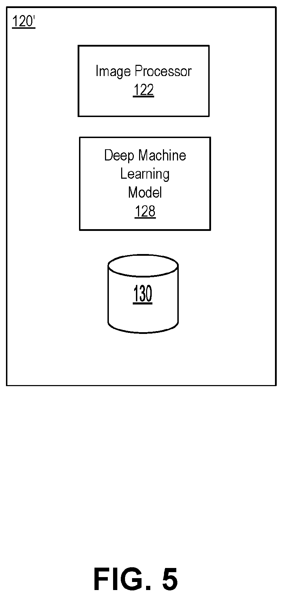

**FIG. 5** is a schematic diagram of a computing device 120', according to an embodiment. In some embodiments, system 100 can include a computing device 120' in place of a computing device 120. As depicted, computing device 120' includes an image processor 122, a deep machine learning model 128, and a electronic datastore 130.

Image processor 122 provides image data of polarized light images to deep machine learning model 128. Optionally, image processor 122 may apply pre-processing to the image data (e.g., normalization) to prepare the data for processing by machine learning model 128.

Deep machine learning model 128 processes the polarized light image data received from image processor 122 to perform one or more of the classification and prediction functions described herein. To this end, deep machine learning model 128 may perform some or all of the functions of classifier 124 and/or predictor 126. For convenience, deep machine learning model 128 may also be referred to as model 128.

In the depicted embodiment, model 128 is configured to generate, for a given specimen 90 imaged in the polarized light image data, a classification metric reflective of a likelihood of the ploidy status of that specimen 90. For example, model 128 may generate a classification metric reflective of the likelihood that the specimen 90 is euploid. Similarly, model 128 may generate a classification metric reflective of the likelihood that the specimen 90 is monoploid, or aneuploid, or the like. In this way, model 128 may function as an image classifier, i.e., processing image data to generate classification metric reflective of a classification of the image data.

Model 128 may utilize data stored in electronic datastore 130. For example, a fully trained model 128 may be instantiated using parameter data stored in electronic datastore 130.

Electronic datastore 110 may implement a conventional relational, non-relational, or object-oriented database, such as Microsoft SQL Server, Oracle, DB2, Sybase, Pervasive, MongoDB, NoSQL, or the like.

In the depicted embodiment, model 128 may implement a CNN that receives polarized light image data at an input layer and provides classification metrics at an output layer. The CNN of model 128 may include a plurality of inner layers. The layers of model 128 may be configured, for example, to apply a plurality of independent convolutions, followed by a non-linearity such as Rectified Linear Unit (ReLU), a sigmoid or the like, with downsampling provided by a pooling operation such as, for example, max-pooling.

In one specific example, given an image with size 224x224x3 (i.e., 224x224 pixels with RGB color channels), 64 convolutions may be applied followed by a max-pooling operation to yield a 112x112x64 output. The convolutions may use a suitably-sized kernel such as, for example, a 3x3 kernel. These steps may be repeated (e.g., in subsequent layers of the CNN), lowering the spatial dimensions at subsequent layers and increasing the number of convolutions used until a layer provides a 1x1x4096 output. This 1x1x4096 output may then be processed (e.g., flattened) via one or more fully connected layers (i.e., dense layers), with a final output (e.g., a classification metric) provided through an output layer of the CNN.

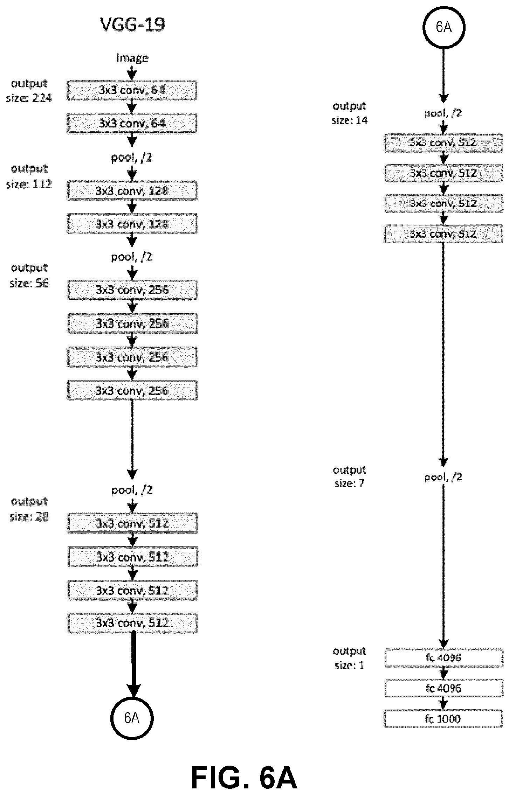

In one embodiment, model 128 may implement a VGG-19 CNN architecture or a VGG-16 architecture, e.g., as described in Simonyan, K., & Zisserman, A. (2014). Very deep convolutional networks for large-scale image recognition. arXiv preprint arXiv:1409.1556. **FIG. 6A** depicts an example VGG-19 architecture which may be implemented by model 128, in an embodiment.

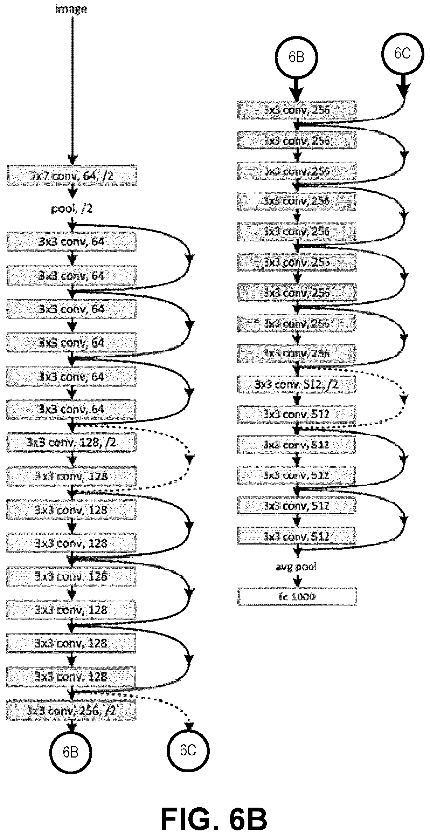

In other embodiments, model 128 may implement other suitable CNN architectures for image classification, such as, for example, ResNet-34, e.g., as described in He, K., Zhang, X., Ren, S., & Sun, J. (2016). Deep residual learning for image recognition. In Proceedings of the IEEE conference on computer vision and pattern recognition (pp. 770-778). ResNet-34 includes residual layers, whereby the input to a convolution is added to its output. **FIG. 6B** depicts an example ResNet-34 architecture which may be implemented by model 128, in an embodiment.

In other embodiments, model 128 may implement other architectures such as ResNet-50 or ResNet-110. Yet other suitable CNN architectures apparent to persons skilled in the art may be implemented at model 128.

Model 128 is trained using suitable labeled training data and a suitable optimization process to minimize a loss function (e.g., a cross-entropy loss). Labelled training data may, for example, include, a plurality of images of a plurality of specimens 90 and a label of the ground truth of ploidy status for each of the specimens 90. Such ground truth may be obtained, for example, from PGT-A screening results for the specimens 90. In some cases, the PGT-A screening results may be supplemented (or corrected, as appropriate) using further ground truths obtained subsequently, such as later during pregnancy or after a birth. Such data may, for example, include a chromosome number indicative of ploidy status. Such further ground truths may, for example, provide improved training data for training model 128.

In some embodiments, model 128 is trained using training data selected based on a particular specimen demographic. For example, the training data may be selected from patients of a certain age range (over 40, or over 45, for example). The trained model can then be used for patients falling in such demographic.

In some embodiments, model 128 is trained using training data for which PGT-A screening results are expected to be particularly reliable (e.g., having a low rate of false positives or false negatives). This may provide improved training data for training model 128.

In some embodiments, model 128 includes a single head, e.g., a classification head for outputting classification metrics. In some embodiments, model 128 includes multiple heads. For example, in some such embodiments, model 128 includes a classification head and also a segmentation head. The segmentation head is configured to identify regions (or segments) of an image that correspond to a particular embryo feature.

In an embodiment, the segmentation head is configured to identify regions of an image that represent an ICM. In another embodiment, the segmentation head is configured to identify regions of an image that represent a TE. In other embodiments, the segmentation head can be configured to identify regions of an image that represent yet other features such as, for example, a zona.

The segmentation head may identify regions of an image representing a particular feature by, for example, calculating for each pixel of the image, a likelihood that the pixel represents a part of that particular feature. For example, segmentation head can generate a metric for each pixel that represents a prediction of whether that pixel represents a part of the ICM. Similarly, segmentation head can generate a metric for each pixel that represents a prediction of whether that pixel represents a part of the TE, or another feature that model 128 is trained to identify.

The segmentation head is trained using suitable labeled training data and a suitable optimization process to minimize a loss function (e.g., a cross-entropy loss). The labelled training data may include, for example, for a plurality of images of a plurality of specimens 90, data for each of the images identifying pixels of that image representing a particular embryo feature.

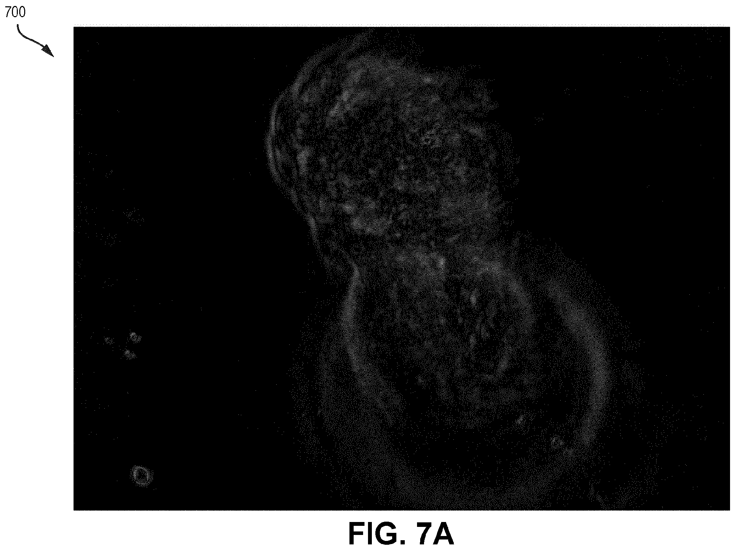

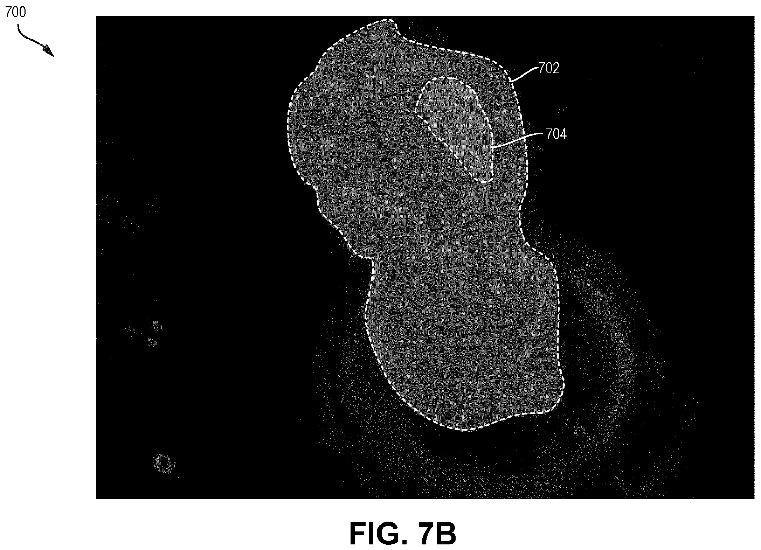

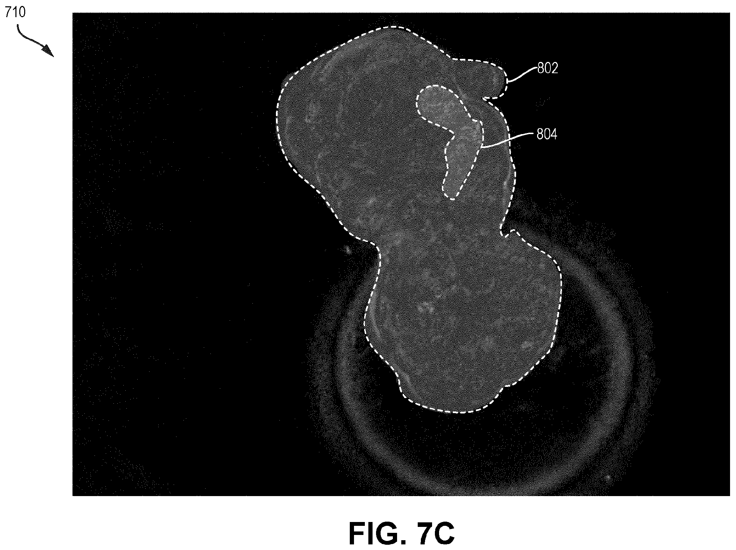

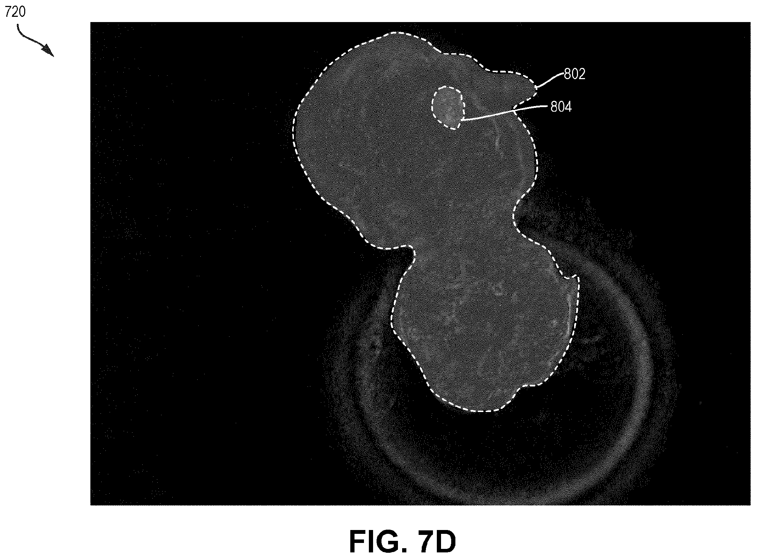

For example, given an image 700 of a specimen 90, as shown in **FIG. 7A****,** desired features of specimen 90 can be labelled to identify pixels of that image representing those features. **FIG. 7B** shows the image 700 of **FIG. 7A** with a labeled pixel region 702 containing pixels that represent the TE and a labeled pixel region 704 containing pixels that represent the ICM. **FIG. 7C** and **FIG. 7D** show, respectively, images 710 and 720 which show different layers of the same specimen 90, e.g., imaged with different focus settings. As depicted, images 710 and 720 have been similarly labelled to identify regions 702 and 704 containing pixels that represent the TE and ICM, respectively. The labelling of pixel regions may be applied manually, e.g., with a suitable labeling tool.

In embodiments providing a model 128 with a classification head and a segmentation head, model 128 is trained by optimizing a total model loss as follows:
$Total model loss = \left(classification loss\right) + A * \left(segmentation loss\right)$ where classification loss is a loss for the classification head and segmentation loss is an auxiliary loss for the segmentation. Both classification loss and segmentation loss may, for example, be a cross-entropy loss. The parameter A is a numeric parameter for tuning the relative weight of the classification loss and the segmentation loss. The value of the parameter A may be selected empirically, e.g., to obtain a value that minimizes classification loss.

As noted, in some embodiments, the segmentation head may be trained to identify an image region that represents an ICM. In such embodiments, this may help model 128 to hone on the image pixels that have the most predictive value by explicitly teaching the model how to identify such pixels. For example, given domain knowledge that suggests the ICM is the region most predictive of an euploid embryo, training the segmentation head to identify pixels of an image that represent the ICM may, in some embodiments, improve performance of a classification head that predicts an euploid status, and result in a lower classification loss.

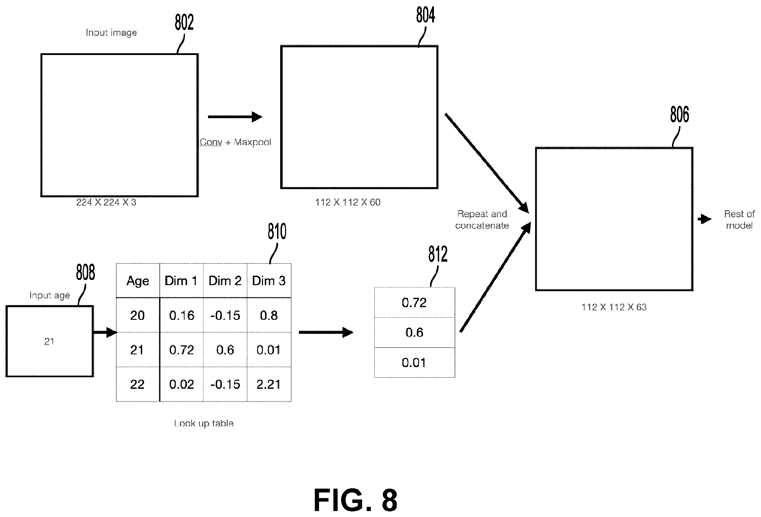

In some embodiments, model 128 may be configured to take into account metadata accompanying the polarized light image data. Such metadata may include, for example, an age of the embryo, an age of one or both parents, or the like. An example of such an embodiment is depicted in **FIG. 8****.**

In the embodiment depicted in **FIG. 8****,** model 128 is configured to take into account metadata that is the mother's age. Model 128 maintain a lookup table 810 (e.g., in electronic datastore 130) that maps each possible age to a corresponding vector. As depicted, each of these vectors has a length of 3, i.e., containing Dim 1, Dim 2, and Dim 3. However, the length of this vector may vary in other embodiments. Prior to training model 128, look-up table 810 may be initialized with random values. During training, the values of look-up table 810 are updated (in concert with the rest of model 128 such as its filter values) to minimize the model's loss function.

During example operation, model 128 may receive both image data for a polarized light image 802 and an input age 808, i.e., an example age 21. Model 128 uses look-up table 810 to retrieve a vector 812 associated with input age 808, i.e., [0.72, 0.6, 0.01]. Vector 812 is then concatenated with the image data with replication of vector 812 corresponding to the dimensionality of the image data. For example, given image data for a polarized light image 802 with example dimensions 224x224x3 (reflecting 3 RGB channels), and a vector 812 with a length of 3, during concatenation, the vector 812 is replicated such that after concatenation the input data is 224x224x6 (3 from RGB channels and 3 from vector 812).

In some embodiments, vector 812 may be concatenated with image data not at an input layer of the CNN of model 128, but rather at an inner layer. For example, as shown in **FIG. 8****,** although polarized light image 802 is provided to an input layer of the CNN of model 128, vector 812 is provided at an inner layer, e.g., after certain convolutions and pooling operations have been applied to polarized light image 802 to transform the data with dimensions 224x224x3 to data with dimensions 112x112x60. Vector 812 is then replicated and concatenated with this transformed data (e.g., as outputted by the preceding layer) to create concatenated data having dimensions 112x112x63. This concatenated data is provided to the subsequent inner layer of the CNN of model 128.

Conveniently, providing vector 812 representing the age metadata at an inner layer of the CNN of model 128 requires fewer replications of vector 812 when dimensionality of the image data has been reduced at such inner layer. This may facilitate more efficient computation.

As noted, image processor 122 may process image data frames from video data captured over a period of time, which may reflect differing focus of specimen 90, with each image data frame thereby reflecting a different layer of specimen 90. Accordingly, in some embodiments, model 128 may be configured to generate a classification metric upon processing image data that includes a single frame reflecting a particular imaged layer of specimen 90. Conversely, in some embodiments, model 128 may be configured to generate a classification metric upon processing a plurality of frames, with each frame reflecting a particular imaged layer of specimen 90. Providing model 128 with additional information regarding specimen 90 in additional frames may improve classification performance.

In an embodiment, model 128 is configured to process a plurality of frames by performing 2D processing in manners described above to produce a representation vector for each frame, and then applying one or more 1D convolutional layers to perform a sequence of convolutions on the representation vectors in a time dimension of the video (corresponding to a spatial dimension of the layers of specimen 90) to generate a classification metric. In another embodiment, model 128 is configured to receive the image data for all the frames in a combined volume having, for example, dimensions 30x224x224x3, where 30 is the number of layers, and 224x224x3 are the dimensions of each frame. A 3D CNN may be applied to this inputted volume to generate a classification metric.

In some embodiments, image processor 122 may automatically select a subset of frames (e.g.., one or more frames) from video data for processing by model 128. For example, image processor 122 may select the frame or frames based on various heuristics or machine learning models configured to provide model 128 with the most useful data for classification. In an embodiment, a frame that includes the most pixels representing the ICM may be automatically selected.

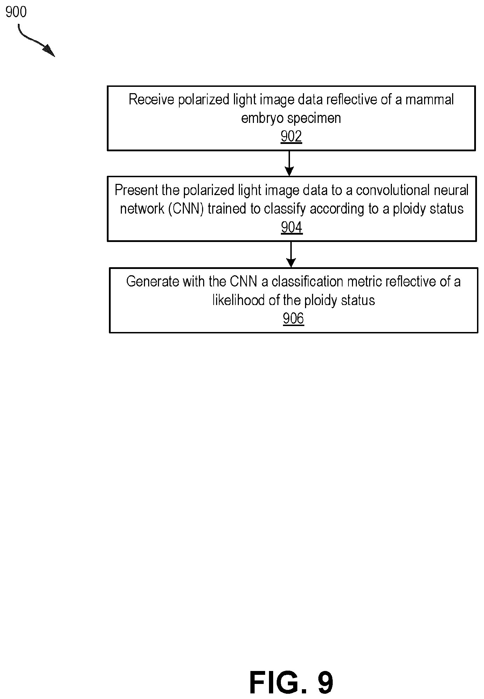

**FIG. 9** is a flow chart of a method 400 for classifying ploidy status, as may be performed at a computing device 120' with a deep machine learning model 128, according to an embodiment. The steps are provided for illustrative purposes. Variations of the steps, omission or substitution of various steps, or additional steps may be considered.

At blocks 902, polarized light image data reflective of a mammal embryo specimen are received, e.g., from image processor 122.

At block 904, the polarized light image data are presented to a CNN of model 128, which is trained to classify according to a ploidy status.

At block 906, a classification metric reflective of a likelihood of the ploidy status is generated with the CNN. The polidy status can include at least one of aneuploidy, monoploidy, or euploidy. The classification metric may be received from a classification head of the CNN. In some embodiments, predictions may be received from the CNN, e.g., from a segmentation head, for each pixel in the image data, whether the pixel represents a particular embryo feature (e.g., an ICM, a TE, a zone, or the like).

It should be understood that blocks 902 to 906 may be performed in a different sequence or in an interleaved or iterative manner.

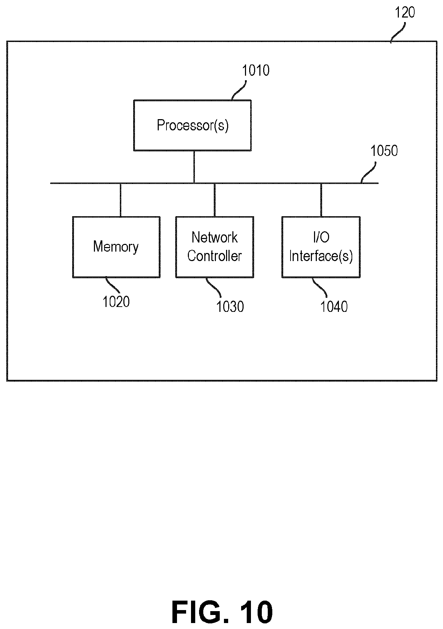

**FIG. 10** is a high-level block diagram of a computing device 120 or computing device 120'. System 100 may be implemented as software and/or hardware, for example, in a computing device 120 or a computing device 120' as illustrated in **FIG. 10****.**

As illustrated, computing device 120 or computing device 120' includes one or more processor(s) 1010, memory 1020, a network controller 1030, and one or more I/O interfaces 1040 in communication over bus 1050.

Processor(s) 1010 may be one or more Intel x86, Intel x64, AMD x86-64, PowerPC, ARM processors or the like.

Memory 1020 may include random-access memory, read-only memory, or persistent storage such as a hard disk, a solid-state drive, cloud storage or the like. Read-only memory or persistent storage is a computer-readable medium. A computer-readable medium may be organized using a file system, controlled and administered by an operating system governing overall operation of the computing device.

Network controller 1030 serves as a communication device to interconnect the computing device with one or more computer networks such as, for example, a local area network (LAN) or the Internet.

One or more I/O interfaces 1040 may serve to interconnect the computing device with peripheral devices, such as for example, keyboards, mice, video displays, and the like. Such peripheral devices may include a display of device 120 or 120'. Optionally, network controller 1030 may be accessed via the one or more I/O interfaces.

Software instructions are executed by processor(s) 1010 from a computer-readable medium. For example, software may be loaded into random-access memory from persistent storage of memory 1020 or from one or more devices via I/O interfaces 1040 for execution by one or more processor(s) 1010. As another example, software may be loaded and executed by one or more processor(s) 1010 directly from read-only memory.

Example software components and data stored within memory 1020 of computing device 120 or computing device 120' may include software to detect spindles and predict ploidy, as described herein, and operating system (OS) software allowing for basic communication and application operations related to computing device 120 or computing device 120'.

Methods 300, 400, and 900 in particular, one or more of blocks 302 to 312, 402 to 406, 902 to 906, respectively, may be performed by software and/or hardware of a computing device such as computing device 120 or computing device 120'.

Memory 1020 may include machine learning code with rules and models such as classifier 124, predictor 126 or machine learning techniques or neural networks such as deep machine learning model 128. The machine learning code can refine classifications and predictions based on learning.

### Example

Thirty women going through a standard in-vitro fertilization (IVF) cycle, who are interested in doing PGT-A after embryo creation and provide consent will be recruited for the study.

Just before performing the trophectoderm embryo biopsy (day 5 or day 6 after ICSI), an expert embryologist will perform the polarized light birefringence imaging exam for each embryo and record the result. The next steps will be the embryo biopsy, freezing the embryo and sending the biopsied cells to the genetic lab. Another polarized light birefringence imaging exam will be performed for each warmed embryo before performing the embryo transfer.

The results of the polarized light birefringence imaging exam will not influence the clinical decisions about which embryo to transfer.

The treatment of all of the participants in this study (excluding the polarized light birefringence imaging exam) will be the same, and not different than the clinic strict standards, as for each other woman treated with IVF.

As in the regular IVF cycle, study participant(s) will come in cycle D3, D7, D10, and D13-14, along with retrieval dates. There will be total of about 5 visits in the clinic, not different from any other IVF cycle.

The biochemical pregnancy rate, clinical pregnancy rate, ongoing pregnancy rate, miscarriage rate, and live birth rate will be tracked for all participants.

It is expected that there will be a correlation between ploidy status by PGT-A and refractive index before freezing (by the polarized light birefringence imaging system).

Embodiments of systems and methods as disclosed herein may be used for the screening of an embryo as a specimen 90 after thaw, using imaging such as birefringence. Conveniently, imaging techniques as disclosed herein may be used to determine viability and ploidy in a non-invasive manner of an embryo, so that a patient may receive an embryo that is predicted to give a good chance of a successful and safe pregnancy.

Of course, the above described embodiments are intended to be illustrative only and in no way limiting. The described embodiments are susceptible to many modifications of form, arrangement of parts, details and order of operation. The disclosure is intended to encompass all such modification within its scope, as defined by the claims.

### References

1. Practice Committees of the American Society for Reproductive Medicine and the Society for Assisted Reproductive Technology. The use of preimplantation genetic testing for aneuploidy (PGT-A): a committee opinion. Fertil Sterility^{®}. 2018;109:429-36.
2. Homer HA, Homer A, Chen C. Preimplantation genetic testing for aneuploidy A ): The biology, the technology and the clinical outcomes. Aust N Z J Obs Gynaecol. 2019;59:317-24.
3. Mastenbroek S, Twisk M, Veen F Van Der, Repping S. Preimplantation genetic screening: a systematic review and meta-analysis of RCTs. Hum Reprod Update. 2011;17:454-66.
4. Bolton H, Graham SJL, Aa N Van Der, Kumar P, Theunis K, Gallardo EF, et al. normal developmental potential. Nat Commun [Internet]. Nature Publishing Group; 2016;7:1-12. Available from: http://dx.doi.org/10.1038/ncomms11165
5. Scott, R.J., Ferry, K., Su, J., Tao, X, Scott, K., Treff, N. Comprehensive chromosome screening is highly predictive of the reproductive potential of human embryos: a prospective, blinded, nonselection study. Fertil & Steril 2012; 97:870-875.
6. Munné S, Nakajima ST, Najmabadi S, Sauer MV, Angle MJ, Rivas JL, Mendieta LV, Macaso TM, Sawarkar S, Nadal A, Choudhary K, Nezhat C, Carson SA, Buster JE. First PGT-A using human in vivo blastocysts recovered by uterine lavage: comparison with matched IVF embryo controls†. Hum Reprod. 2020;35:70-80.
7. Victor AR, Tyndall JC, Brake AJ, Lepkowsky LT. One hundred mosaic embryos transferred prospectively in a single clinic : exploring when and why they result in healthy pregnancies. Fertil Steril 2019;111:280-293.
The present invention may also be described or defined in accordance with the following clauses:
1. A computer-implemented system for classifying ploidy status, the system comprising:
   a processor; and
   a memory in communication with the processor, the memory storing instructions that, when executed by the processor, cause the processor to:
      receive polarized light image data reflective of a mammal embryo specimen;
      present the polarized light image data to a convolutional neural network (CNN) trained to classify specimens according to a ploidy status; and
      generate with the CNN a classification metric reflective of a likelihood of the ploidy status.
2. The system of clause 1, wherein the ploidy status includes at least one of aneuploidy, mosaicism, or euploidy.
3. The system of clause 1 or clause 2, wherein the classification metric is received from a classification head of the CNN, and the CNN further includes a segmentation head configured to predict, for a given pixel in the image data, whether the pixel represents a particular embryo feature.
4. The system of clause 3, wherein the CNN is trained using a loss function that includes a classification loss for the classification head, and a segmentation loss for the segmentation head, and the loss function includes a relative weight of the classification loss and segmentation loss.
5. The system of clause 3 or clause 4, wherein the particular of embryo feature is an inner cell mass, a trophectoderm, or a zona.
6. The system of any one of clauses 1 to 5, wherein the polarized light image data includes a frame reflecting a particular imaged layer of the mammal embryo specimen.
7. The system of any one of clauses 1 to 5, wherein the polarized light image data includes a plurality of frames, each reflecting a particular imaged layer of the mammal embryo specimen.
8. The system of clause 7, wherein the CNN includes an inner layer configured to produce a plurality of representation vectors, each corresponding to one of the plurality of frames, and the representation vectors are provided to a 1D convolutional layer of the CNN.
9. The system of clause 7, wherein the CNN is a 3D convolutional neural network and the polarized image data is organized as a volume including the plurality of frames.
10. The system of any one of clauses 1 to 9, wherein the instructions, when executed by the processor cause the processor to: provide metadata of the mammal embryo specimen to the CNN.
11. The system of clause 10, wherein the metadata is provided to an inner layer of the CNN.
12. The system of clause 11, wherein the metadata is concatenated to the output of a layer preceding the inner layer.
13. The system of any one of clauses 10 to 12, wherein the instructions, when executed by the processor cause the processor to: maintain a look-up table for mapping values of the metadata to values trained with the CNN.
14. The system of any one of clauses 10 to 13, wherein the metadata includes a patient's age.
15. The system of any one of clauses 1 to 14, wherein the mammal is a human.
16. The system of any one of clauses 1 to 15, wherein the instructions, when executed by the processor cause the processor to generate the polarized light image data upon determining birefringence properties of the mammal embryo specimen.
17. A computer-implemented method for classifying ploidy status, the method comprising:
   receiving polarized light image data reflective of a mammal embryo specimen;
   presenting the polarized light image data to a convolutional neural network (CNN) trained to classify according to a ploidy status; and
   receiving from the CNN a classification metric reflective of a likelihood of the ploidy status.
18. A computer-implemented system comprising:
   an image sensor;
   a processor in communication with the image sensor; and
   a memory in communication with the processor, the memory storing instructions that, when executed by the processor cause the processor to:
      receive, from the image sensor, image data representing emerging polarized light that has traversed a specimen;
      determine birefringence properties of the specimen based at least in part on the image data;
      generate a polarized light image representative of the specimen based at least in part on the birefringence properties;
      classify features of the polarized light image using a classifier;
      identify features of the polarized light image as mitotic spindles;
      determine mitotic activity of the specimen based at least in part on the identified mitotic spindles; and
      predict a ploidy status of the specimen based on the mitotic activity.
19. The system of clause 18, wherein the memory stores further instructions that, when executed by the processor cause the processor to: determine whether the mitotic activity is below a predetermined threshold, and when the mitotic activity is below the predetermined threshold the ploidy status of the specimen is predicted to be euploid.
20. The system of clause 18 or clause 19, wherein the mitotic activity of the specimen is determined based at least in part on a number of the identified mitotic spindles.
21. The system of any one of clauses 18 to 20, wherein the memory stores further instructions that, when executed by the processor cause the processor to: determine geometric shapes of the identified mitotic spindles; and the mitotic activity of the specimen is determined based at least in part on the geometric shapes of the identified mitotic spindles.
22. The system of any one of clauses 18 to 21, wherein the memory stores further instructions that, when executed by the processor cause the processor to: identify features of the polarized light image as an inner cell mass (ICM) and a trophectoderm (TE); determine locations of the identified mitotic spindles as in the ICM or in the TE; and the mitotic activity of the specimen is determined based at least in part on the locations of the identified mitotic spindles.
23. The system of any one of clauses 18 to 22, wherein the specimen is from a mammal embryo.
24. The system of clause 23, wherein the mammal is a human.
25. A method of selecting for implantation an embryo of a mammal egg fertilized in vitro comprising:
   quantifying mitotic activity in the trophectoderm (TE) and/or the inner cell mass (ICM) of an embryo based on optical inspection thereof; and
   selecting an embryo that has mitotic activity below a predetermined threshold.
26. The method of clause 25, wherein an embryo having mitotic activity below the predetermined threshold is predicted to be euploid.
27. The method of clause 25 or clause 26, wherein the optical inspection comprises imaging the embryo using polarized light with a microscope equipped with a retardance filter, wherein a detected degree of retardance corresponds to the mitotic activity.
28. The method of any one of clauses 25 to 27, wherein the mitotic activity is based at least in part on the number of mitotic spindles identified in the ICM.
29. The method of any one of clauses 25 to 28, wherein the mitotic activity is based at least in part on the number of mitotic spindles identified in the TE.
30. The method of clause 28, wherein the predetermined threshold is zero mitotic activity in the ICM.
31. The method of any one of clauses 25 to 30, wherein the mitotic activity is based at least in part on the total number of mitotic spindles in the TE and ICM.
32. The method of any one of clauses 25 to 31, wherein the mitotic activity is a weighted average of the number of mitotic spindles in the ICM and the total number of mitotic spindles in the TE and ICM.
33. The method of any one of clauses 25 to 32, wherein the optical inspection is performed between 2 days and 10 days after fertilization of the specimen.
34. The method of clause 33, wherein the optical inspection is performed at 5 days after fertilization of the specimen.
35. The method of any one of clauses 25 to 34, further comprising implanting the selected embryo.
36. The method of clause 35, wherein the embryo is not frozen prior to implantation.
37. The method of any one of clauses 25 to 36, wherein the mammal is a human.
38. The method of clause 37, wherein the threshold is determined by a patient.
39. A method for optimizing the likelihood of a live birth after implantation of a human egg fertilized in vitro comprising: performing the method of clause 25 on a plurality of embryos developed after in vitro fertilization and selecting an embryo that is predicted to be euploid for implantation.
40. The method of clause 39 comprising selecting the embryo having the highest probability of being euploid for implantation.
41. A tool for assisting a human patient undergoing in vitro fertilization treatment in deciding whether to proceed with implantation of an embryo resulting from a human egg fertilized in vitro, the tool comprising an indicia of the probability of the embryo being euploidy and/or aneuploidy based on the number of mitotic spindles in the **TE** and/or the ICM as observed by an optical inspection method at the blastocyst stage.

## Claims

1. A method of selecting for implantation an embryo of a mammal egg fertilized in vitro comprising:
quantifying mitotic activity in the trophectoderm (TE) and/or the inner cell mass (ICM) of an embryo based on optical inspection thereof; and
selecting an embryo that has mitotic activity below a predetermined threshold.

2. The method of claim 1, wherein an embryo having mitotic activity below the predetermined threshold is predicted to be euploid.

3. The method of claim 1 or claim 2, wherein the optical inspection comprises imaging the embryo using polarized light with a microscope equipped with a retardance filter, wherein a detected degree of retardance corresponds to the mitotic activity.

4. The method of any one of claims 1 to 3, wherein the mitotic activity is based at least in part on the number of mitotic spindles identified in the ICM

5. The method of any one of claims 1 to 4, wherein the mitotic activity is based at least in part on the number of mitotic spindles identified in the TE.

6. The method of claim 1, wherein the predetermined threshold is zero mitotic activity in the ICM.

7. The method of any one of claims 1 to 6, wherein the mitotic activity is based at least in part on the total number of mitotic spindles in the TE and ICM.

8. The method of any one of claims 1 to 7, wherein the mitotic activity is a weighted average of the number of mitotic spindles in the ICM and the total number of mitotic spindles in the TE and ICM.

9. The method of any one of claims 1 to 8, wherein the optical inspection is performed between 2 days and 10 days after fertilization of the specimen.

10. The method of claim 9, wherein the optical inspection is performed at 5 days after fertilization of the specimen.

11. The method of any one of claims 1 to 10, wherein the mammal is a human.

12. The method of claim 11, wherein the threshold is determined by a patient.

13. A method for optimizing the likelihood of a live birth after implantation of a human egg fertilized in vitro comprising: performing the method of claim 1 on a plurality of embryos developed after in vitro fertilization and selecting an embryo that is predicted to be euploid for implantation.

14. The method of claim 13 comprising selecting the embryo having the highest probability of being euploid for implantation.
